# EUROPÄISCHE PATENTSCHRIFT

(11) **EP 3 035 976 B1**
(45) Veröffentlichungstag und Bekanntmachung des Hinweises auf die Patenterteilung: **02.10.2019**
(21) Anmeldenummer: 14752280.9
(22) Anmeldetag: 14.08.2014
(51) Int. Cl.: A61M 1/16, A61B 5/00, A61M 1/36, A61B 5/1455, G01N 21/00, G01N 33/06, G01N 33/49

(54) **EINWEGARTIKEL FÜR DIE DIALYSEBEHANDLUNG UND DIALYSEGERÄT**
DISPOSABLE ARTICLE FOR DIALYSIS TREATMENT AND DIALYSIS MACHINE
ARTICLE À USAGE UNIQUE POUR LE TRAITEMENT PAR DIALYSE ET APPAREIL DE DIALYSE

(30) Priorität: 23.08.2013 DE 102013014097
(43) Veröffentlichungstag der Anmeldung: 29.06.2016
(73) Patentinhaber: Fresenius Medical Care Deutschland GmbH, 61352 Bad Homburg (DE)
(72) Erfinder: GLASER, Benedict, 97421 Schweinfurt (DE)
(74) Vertreter: Herrmann, Uwe
(86) Internationale Anmeldenummer: PCT/EP2014/002246
(87) Internationale Veröffentlichungsnummer: WO 2015/024647

(56) Entgegenhaltungen:
- WO-A1-2005/077262
- WO-A1-2009/153331
- WO-A1-2010/108714
- WO-A1-2011/079907
- WO-A2-01/89599
- WO-A2-2012/095301
- DE-A1-102008 010 948
- DE-A1-102010 038 923
- US-A1- 2007 007 184
- US-A1- 2009 088 683
- US-A1- 2012 316 799

## Beschreibung

Die Erfindung betrifft einen Einwegartikel zur Verwendung in der Dialysebehandlung und eine Dialysemaschine mit einem solchen Einwegartikel.

An flüssigkeitsführenden Leitungen in der Dialyse ist eine Mehrzahl von Sensoren notwendig, um eine sichere Blutbehandlung zu gewährleisten. Mehrere Zustandsgrößen des Blutes und der Behandlungsflüssigkeiten werden während der Dialysebehandlung laufend registriert und die gewonnenen Daten in einer Prozessoreinheit ausgewertet. Insbesondere werden Temperatur, Druck, Flüssigkeitsniveau, Leitfähigkeit, akustische Eigenschaften gemessen, um eine Aussage über die Patientensicherheit machen zu können und im Zweifelsfall einen Alarm oder einen Behandlungsabbruch auszulösen.

In der Regel wird das Blut in der Hämodialyse durch einen extrakorporalen Kreislauf geführt, der als Einwegartikel ausgestaltet ist. Der extrakorporale Kreislauf ist aufgebaut aus Schlauchleitungen, Filtermodulen, Konnektoren, Tropfkammern und weiteren Elementen. An bisher bekannten Systemen zur Dialysebehandlung sind die Sensoren Teile der Behandlungsmaschine. Diese müssen mit entsprechenden Teilen des extrakorporalen Kreislaufs in Eingriff gebracht werden, damit Messungen durchgeführt werden können. Dies erschwert die Aufrüstung der Dialysemaschine.

Darüber hinaus geht das derzeitige Entwicklungsbestreben dahin, den extrakorporalen Kreislauf komplexer zu konstruieren. Es werden im Stand der Technik zahlreiche Blutbehandlungskassetten beschrieben, mit denen mehr oder weniger versucht wird, den extrakorporalen Kreislauf zu miniaturisieren, um Material und Ressource einzusparen. Entsprechend erschwert sich durch die zunehmende Komplexität des extrakorporalen Kreislaufs konstruktiv die Sensorankopplung zwischen Dialysemaschine und dem als Disposable ausgebildeten extrakorporalen Kreislauf.

Die US 2007/007184 A1 offenbart einen Einwegartikel für die Dialysebehandlung, der alle Merkmale des Oberbegriffs des Anspruchs 1 aufweist.

Die Erfindung schlägt vor diesem Hintergrund einen Einwegartikel zur Verwendung in der Dialysebehandlung nach Anspruch 1 vor, der wenigstens einen Sensor zur Ermittlung eines die Dialyse betreffenden Messwertes aufweist.

Der Begriff eines die Dialyse betreffenden Messwertes ist im Rahmen der vorliegenden Erfindung breit zu interpretieren und umfasst alle Zustandsgrößen oder Parameter des Dialysats oder Bluts, die irgendwo im Einwegartikel gemessen werden können. Auf bevorzugte Ausführungsformen wird an späterer Stelle eingegangen. Kerngegenstand der Erfindung ist daher, dass das Disposable selbst einen vollständigen Sensor aufweist, und der jeweilige Sensor oder ein Teil des Sensors daher nicht auf der Maschinenseite angeordnet sind. Daher ist die Aufrüstung der Dialysemaschine erleichtert und die Messung erfolgt direkt und akkurat, d.h. es befindet sich kein potentiell störendes Disposable zwischen Sensor und Messmedium Blut oder Dialysat.

Nach der Erfindung weist der Einwegartikel ferner wenigstens einen Sender auf, der so ausgebildet ist, dass er eine drahtlose Übertragung des Messwertes an einen außerhalb des Einwegartikels befindlichen Empfänger ermöglicht. Derartige Systeme können also das Messsignal über Funk an einen Empfänger übertragen. Es entstehen viele Vorteile. Unter anderem ist das Messdatenerfassungssystem örtlich unabhängig und es ist eine Messdatenerfassung am Patienten oder an einer schwer zugänglichen Stelle im Dialysegerät möglich. Die Notwendigkeit eines Kabels entfällt, was eine einfache Montage und eine Aussparung von Bauraum für ein Kabel bedingt.

Nach der Erfindung weist der Einwegartikel ferner wenigstens einen thermoelektrischen Energiewandler zur Bereitstellung elektrischer Energie auf. Diese Verwendung von technischen Systemen zur Energiegewinnung aus der Umwelt wird auch als Energy Harvesting bezeichnet. In der Dialysetechnik stehen als Energiequellen beispielsweise die Temperaturdifferenz zwischen Dialysat bzw. Blut und der Umgebung (thermische Energie) oder Druckänderungen oder Bewegungen, z. B. die durch eine Peristaltikpumpe erzeugte Pulsation oder Druckänderung (kinematische Energie, mechanische Energie) zur Verfügung. Ferner kann beispielsweise elektromagnetische Energie eingebracht werden. Dies ermöglicht einen autarken Betrieb des Sensors und/oder des Senders und hat gegenüber beispielsweise einer Batterie oder einem Akku Vorteile ggf. geringerer Kosten und geringeren Platzbedarfs. Ferner enthalten eine Batterie oder ein Akku oft Giftstoffe, die in der Behandlung und der Entsorgung des Disposables problematisch sind. Des Weiteren haben Batterien oder Akkus eine begrenzte Haltbarkeit bei längerer Lagerung oder Einsatzdauer.

Im Einwegartikel können mehrere Sensoren und/oder Sender und/oder Energiewandler vorhanden sein. Bevorzugt ist, dass jeder Sensor mit einem Sender und einem Energiewandler innerhalb des Disposables in Verbindung, beispielsweise Kabelverbindung steht oder eine bauliche Einheit bildet. Bevorzugt ist ferner, dass jeder Sender mit einem Energiewandler innerhalb des Disposables in Verbindung, beispielsweise Kabelverbindung steht oder eine bauliche Einheit bildet.

Nach der Erfindung handelt es sich bei dem Energiewandler um einen thermoelektrischen Energiewandler und vorzugsweise ein Peltier-Element. Ein thermoelektrisches Element nützt einen Temperaturunterschied, beispielsweise den Temperaturunterschied zwischen dem im Einwegteil fließenden oder befindlichen Blut bzw. Dialysat und der Umgebung, beispielsweise unter Ausnutzung des Seebeck-Effekts, zur Erzeugung elektrischer Energie. Solche Elemente sind miniaturisierbar und einfach und billig herstellbar.

In einem illustrativen Beispiel ist der Energiewandler ausgebildet, um kinematische oder mechanische Energie in elektrische Energie umzuwandeln. Beispielsweise können derartige Energiewandler ausgebildet sein, um auf Basis von Druckänderungen oder Bewegungen, z. B. durch eine Pumpe, ggf. Peristaltikpumpe erzeugte Pulsation oder Bewegung des im Einwegteil fließenden oder befindlichen Bluts bzw. Dialysats elektrische Energie zu erzeugen.

In einem weiteren illustrativen Beispiel ist der Energiewandler ausgebildet, um elektromagnetische Energie zu empfangen und in elektrische Energie umzuwandeln. Elektromagnetische Energie kann drahtlos übertragen werden. Beispielsweise kann maschinenseitig ein Sender für elektromagnetische Energie vorhanden sein, die dann am Disposable durch den entsprechenden Energiewandler in elektrische Energie umgewandelt wird.

Auch mehrere unterschiedliche Arten von Energiewandlern können an einem erfindungsgemäßen Einwegteil zur Anwendung kommen.

Nach der Erfindung handelt es sich bei dem Sensor um einen Drucksensor. Selbstverständlich sind bei nicht von der Erfindung umfassten Umsetzungen auch weitere Sensoren denkbar, die sich zur Bestimmung einer Zustandsgröße oder eines Parameters des Dialysats oder Bluts eignen. Bevorzugt kommen solche Sensoren zum Einsatz, die mit elektrischer Energie betrieben werden können und einen niedrigen Energieverbrauch aufweisen. Beispiele umfassen einen kapazitiven Drucksensor oder ein Widerstandsthermometer. Auch mehrere unterschiedliche Arten von Sensoren können an einem erfindungsgemäßen Einwegteil zur Anwendung kommen.

Nach der Erfindung handelt es sich bei dem Einwegartikel um einen extrakorporalen Blutkreislauf für die Hämodialyse, der eine Blutleitung mit einer arteriellen und einer venösen Leitung, und vorzugsweise einen Dialysator und eine Dialysatleitung umfasst. Im Rahmen der vorliegenden Erfindung wird der Begriff Hämodialyse als Oberbegriff verwendet, unter den die Hämodialyse im engeren Sinn, die Hämodiafiltration und die Hämofiltration fällt. Die Blutleitung und die Dialysatleitung können beispielsweise als Schlauchleitungen ausgebildet sein. Ferner ist ein Kassettensystem denkbar, wobei die Blutleitung und die Dialysatleitung als Kanäle in der Kassette ausgebildet sind. Das Disposable kann ferner Katheter zum Anschluss der arteriellen und venösen Leitung an den Patienten aufweisen.

Im als Disposable ausgebildeten extrakorporalen Blutkreislauf, der nicht von der Erfindung umfasst ist, kann der Sensor beispielsweise an einer oder mehreren der folgenden Stellen angeordnet sein:
- in der arteriellen Leitung des extrakorporealen Blutkreislaufs;
- in der venösen Leitung des extrakorporealen Blutkreislaufs;
- in der Dialysatleitung stromaufwärts des Dialysators;
- in der Dialysatleitung stromabwärts des Dialysators;
- auf der Blutseite des Dialysators, gegebenenfalls eingangsseitig oder ausgangsseitig; oder
- auf der Dialysatseite des Dialysators, gegebenenfalls eingangsseitig oder ausgangsseitig.

Gemäß einem illustrativen Beispiel ist ein Drucksensor in der venösen Leitung des extrakorporalen Blutkreislaufs angeordnet. Dadurch kann eine Überwachung des venösen Drucks am venösen Katheter erreicht werden. Ein Vorteil in dieser Ausführungsform ist die akkurate Überwachung durch eine Messung unmittelbar am Blut, das Fehlen von störenden Kabeln und die fehlende Relevanz des Abstandes zwischen Patient und Dialysegerät.

In einer Ausführungsform ist ein thermoelektrischer Energiewandler in der venösen Leitung des extrakorporalen Blutkreislaufs und vorzugsweise nahe des Katheters angeordnet. So kann und die Körperwärme des Patienten zur Energiegewinnung genützt werden.

In einer Ausführungsform sind im extrakorporalen Blutkreislauf mindestens zwei Drucksensoren so angeordnet, dass der Transmembrandruck am Dialysator gemessen werden kann. Eine denkbare Anordnung umfasst in diesem Zusammenhang das Vorhandensein von Drucksensoren auf der Dialysatseite und Blutseite des Dialysators, ggf. einseitig oder jeweils eingangsseitig und ausgangsseitig. Eine weitere denkbare Anordnung umfasst das Vorhandensein von Drucksensoren in der arteriellen und/oder venösen Blutleitung sowie in der Dialysatleitung stromaufwärts und/oder stromabwärts des Dialysators.

In einer Ausführungsform sind am Dialysator in Längsrichtung mindestens zwei Drucksensoren so angeordnet, dass der Druckverlauf in Längsrichtung des Dialysators gemessen werden kann. Dies ermöglicht ggf. eine Steigung der Dialyseeffektivität.

Nach einem illustrativen Beispiel befinden sich in der Dialysatleitung, der Blutleitung und/oder dem Dialysator ein oder mehrere Temperatursensoren. So kann die Temperatur akkurat überwacht werden. Bevorzugt ist beispielsweise eine Anordnung eines Temperatursensors auf der Dialysatseite und ggf. ferner auf der Blutseite des Dialysators. So kann der Wärmeeintag ins Blut direkt am Dialysator bestimmt werden und es erfolgt keine Verfälschung durch eventuelle Temperaturverluste am Schlauch. Dies kann zu einer Verbesserungen im Temperaturmanagement am Dialysator genützt werden. Die gesamte Energiebilanz kann direkt am Dialysator aufgenommen werden, sodass der thermische Wirkungsgrad des Wärmetauschers Dialysator ohne systematische Messfehler bestimmt werden kann.

Gemäß einem illustrativen Beispiel handelt es sich bei dem Einwegartikel um einen Behälter für ein Dialysat oder ein Konzentrat zur Herstellung eines Dialysats. Der Einwegartikel kann in dieser Ausführungsform also beispielsweise ein an eine Dialysemaschine anzuschließender Vorratsbehälter sein.

Gemäß einem weiteren illustrativen Beispiel handelt es sich bei dem Sensor um einen Füllstandssensor. Mit Hilfe eines direkt am als Disposable ausgebildeten Behälters angeordneten Füllstandssensors kann der Füllstand akkurat bestimmt werden, ohne dass ein externer Sensor benötigt wird. Der Füllstandssensor kann beispielsweise so ausgebildet sein, dass der Füllstand über eine Druckmessung oder Leitfähigkeitsmessung ermittelt wird.

Alternativ oder zusätzlich ist an einem derartigen Behälter auch ein Leitfähigkeitssensor denkbar, beispielsweise zur Ermittlung der Zusammensetzung des Dialysats bzw. des Konzentrats. Ferner ist auch ein Temperatursensor denkbar. Dieser kann im Beutel angeordnet sein und der Ermittlung der Temperatur bei einer Hitzesterilisation des Dialysats bzw. Konzentrats oder bei einer Reinigung des Behälters. Dieser kann auch in einer zum Beutel gehörigen Ablaufleitung angeordnet sein, um die Temperatur des Dialysats zu bestimmen.

Die Erfindung betrifft ferner ein Dialysegerät mit einem erfindungsgemäßen Einwegartikel. Dieses Dialysegerät kann beispielsweise einen Empfänger für Signale von im Einwegartikel angeordneten Sendern aufweisen. Ferner kann es nach einer nicht von der Erfindung umfassten Modifikation beispielsweise eine Strahlungsquelle zur Abgabe von elektromagnetischer Energie an einen im Einwegartikel angeordneten Energiewandler für elektromagnetische Energie aufweisen.

Ferner ist von der Erfindung auch der Fall umfasst, in dem die autarke Messeinheit bestehend aus Sensor, Sender und Energiewandler maschinenseitig angeordnet ist.

Die Erfindung betrifft dementsprechend ein Dialysegerät mit einem Einwegartikel, der nach einem der Ansprüche 1-5 ausgebildet ist.

Auch maschinenseitig ist es vorteilhaft, das Messsignal über Funk an einen Empfänger zu übertragen und einen autarken Betrieb des Sensors an beliebigen Stellen im Gerät zu ermöglichen, wodurch unter anderem die Notwendigkeit zur Verbauung einiger Kabel entfällt.

Energiewandler und/oder Sensor können dabei in verschiedenen Ausführungsformen so ausgebildet sein und miteinander in Verbindung stehen, wie dies oben im Zusammenhang mit dem erfindungsgemäßen Disposable beschrieben wurde.

In einer Ausführungsform weist das Dialysegerät ein Hydrauliksystem zur Förderung von Dialysat auf und der Sensor ist an einer Stelle dieses Hydrauliksystems angeordnet. Das Konzept der autarken Messdatenerfassung kann in diesem Zusammenhang auf jede Messstelle in der maschinenseitigen Hydraulik übertragen werden. Gegebenenfalls ist ferner auch der Energiewandler an einer oder dieser Stelle des Hydrauliksystems angeordnet. Er kann beispielsweise die Fluidströmung oder die erhöhte Temperatur im Hydrauliksystem zur Energiegewinnung nützen. Ferner kann auch der Sender an einer oder dieser Stelle im Hydrauliksystem angeordnet sein. Der Sender kann aber auch außerhalb des Hydrauliksystems angeordnet sein und mit Sensor und Sender in Verbindung stehen. Die Lagebezeichnung *"am"* Hydrauliksystem benennt einen solchen Kontakt mit Fluid oder Aktoren des Hydrauliksystems, dass Messdaten durch diesen Kontakt erfasst oder Energie durch diesen Kontakt gewonnen werden kann. Die Lagebezeichnung *"außerhalb"* des Hydrauliksystems benennt ein Fehlen eines solchen Kontakts.

Gemäß einem illustrativen Beispiel weist das Dialysegerät eine Wasseraufbereitungsanlage für die Dialysatherstellung auf und der Sensor ist an einer Stelle dieser Wasseraufbereitungsanlage angeordnet. Ferner ist denkbar, dass das Dialysegerät eine Wasseraufbereitungsanlage für die Dialysatherstellung aufweist oder mit einer solchen in Verbindung steht und der Sensor an einer Stelle dieser Wasseraufbereitungsanlage angeordnet ist.

Ferner beschrieben wird eine Wasseraufbereitungsanlage für die Dialysatherstellung mit wenigstens einem Sensor zur Ermittlung wenigstens eines die Dialyse betreffenden Messwertes, wenigstens einem Sender, der eine drahtlose Übertragung des Messwertes an wenigstens einen Empfänger ermöglicht, und wenigstens einem Energiewandler zur Bereitstellung vorzugsweise elektrischer Energie an den Sensor und den Sender.

Energiewandler und/oder Sensor können dabei in verschiedenen Ausführungsformen so ausgebildet sein und miteinander in Verbindung stehen, wie dies oben im Zusammenhang mit dem erfindungsgemäßen Disposable beschrieben wurde.

In der Dialysetechnik werden Wasseraufbereitungsanlagen für die Dialysatherstellung verwendet. Ein autarkes Sensorsystem kann beispielsweise zur Temperaturüberwachung während einer in derartigen Systemen regelmäßig durchgeführten Hitzesterilisation verwendet werden.

Der Sensor ist an einer Stelle des wasserführenden Systems dieser Wasseraufbereitungsanlage angeordnet. Gegebenenfalls ist ferner auch der Energiewandler an einer oder dieser Stelle des wasserführenden Systems angeordnet. Er kann beispielsweise die erhöhte Temperatur während der Hitzesterilisierung zur Energiegewinnung nützen.

Weitere Einzelheiten und Vorteile ergeben sich aus den im Folgenden diskutierten Figuren und Ausführungsbeispielen. In den Figuren zeigen:
- Figur 1:: eine Darstellung der Messung des venösen Blutdrucks direkt am Gefäßzugang anhand eines erfindungsgemäßen Disposables;
- Figur 2:: eine Draufsicht und Schnittdarstellung der Messung des venösen Blutdrucks direkt am Gefäßzugang anhand eines erfindungsgemäßen Disposables;
- Figur 3:: eine Darstellung der Messung von Druck und Temperatur direkt am Dialysator eines Disposables;
- Figur 4:: eine Detailansicht des disposableseitigen Dialysators aus Figur 3;
- Figur 5:: eine Darstellung der Messung von Füllstand und Temperatur direkt an einem Einwegbehälter für Dialsyat;
- Figur 6:: eine schematische Darstellung von Messung, Energiewandlung und Funktechnologie zum Einsatz in einem erfindungsgemäßen Disposable;
- Figur 7:: eine Darstellung der Energiewandlung zum Einsatz in einem erfindungsgemäßen Disposable; und
- Figur 8:: eine Abbildung eines thermoelektrischen Energiewandlers zum Einsatz in einem erfindungsgemäßen Disposable.

### Ausführungsbeispiel 1:

### Messen des venösen Blutdrucks direkt am Gefäßzugang

Der Blutdruck soll direkt am venösen Katheter disposableseitig gemessen werden können.

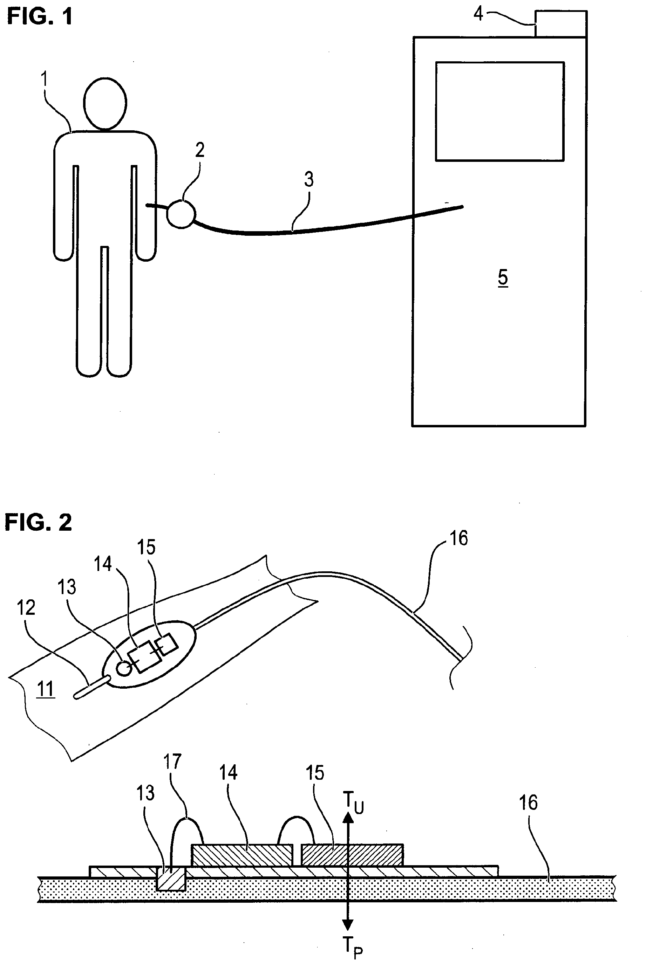

Der Sensor ist direkt am venösen Katheter des als Disposable ausgebildeten extrakorporalen Blutkreislauf umfassend eine Blutleitung mit einer arteriellen und einer venösen Leitung, einen Dialysator und eine Dialysatleitung angeordnet. Dies ist in Figuren 1 und 2 zu erkennen.

In der Darstellung gemäß Figur 1 zeigt Bezugszeichen 1 den Patienten, an dem eine Dialysebehandlung durchgeführt wird. An der venösen Blutrückleitung 3 befindet sich ein Sender 2 zur drahtlosen Übermittlung von Daten an die Empfangseinheit 4, die intern oder extern an der Dialysemaschine 5 angeordnet ist.

In Figur 2 ist der nahe dem Patienten befindliche Teil des Disposables in einer derartigen Anordnung in größerem Detail gezeigt, und zwar links oben in der Figur in einer Draufsicht und rechts unten in der Figur in einer Schnittdarstellung. Mit dem Bezugszeichen 11 ist der Arm des Patienten dargestellt. Die venöse Blutrückleitung 16 ist anhand des Gefäßzugangs 12 mit der Blutbahn des Patienten 11 in Verbindung. Bei dem Gefäßzugang 12 kann es sich beispielsweise um eine Nadel oder einen Katheter handeln. Direkt am Gefäßzugang 12 befindet sich ein disposableseitig angeordneter Drucksensor 13, der den absoluten Druck P_{venös} des Blutes in der venösen Blutrückleitung 16 direkt vor der Rückführung an den Patienten 11 misst. Mit Bezugszeichen 15 ist ein thermoelektrisches Peltier-Element bezeichnet, das den Temperaturunterschied zwischen Umgebungstemperatur T_{U} (beispielsweise 20°C) und der Körperttemperatur des Patienten T_{P} (37°C) zur Erzeugung von elektrischer Energie nützt. Mit Bezugszeichen 14 ist eine Sendeeinheit bezeichnet, die die Messdaten des Drucksensors 13 an einen maschinenseitig angeordneten Empfänger 4 übermittelt. Der Sender 14, der Drucksensor 13 und der thermoelektrische Energiewandler 15 stehen über elektrische Verbindungen 17 in Verbindung. Anhand dieser elektrischen Verbindungen 17 werden der Sender 14 und der Drucksensor 13 mit elektrischer Energie vom Energiewandler 15 versorgt. Ferner gelangen die Messdaten des Drucksensors 13 anhand dieser elektrischen Verbindungen 17 zum Sender 14. Sowohl der Drucksensor 13 als auch der Sender 14 und der Energiewandler 15 sind Teil des Disposables.

Als Sensor wird der kapazitive Drucksensor SCB10H der Fa. VTI verwendet, der einen sehr niedrigem Energieverbrauch hat. Selbstverständlich können auch andere geeignete Drucksensoren zum Einsatz kommen.

Die Anordnung des Sensors direkt am venösen Katheter hat die Vorteile einer sehr kurzen Reaktionszeit bei Diskonnektion der venösen Nadel und einer fehlenden Beeinflussung der Messgröße durch die Schlauchleitung oder Änderungen der Schlauchposition.

Als Sender für die Daten des Sensors wird der der ebenfalls am Disposable angeordnete Drahtlossender STM 312 (868 MHz) der Fa. Enocean verwendet. Alternativ könnte beispielsweise auch der Drahtlossender STM 312C (868 MHz) oder ECT 310 der Fa. Enocean verwendet werden. Als Energiequelle für Sensor und Sender dient ein Standard-Peltierelement in Kombination mit EnOcean Ultra Low Power DC/DC-Wandler, das unter Ausnutzung des Seebeck-Effekts und der Temperaturdifferenz zwischen dem Blut (in der Regel > 35°C) und der Umgebung (in der Regel < 25°C) als Stromquelle dient. Es ist daher keine externe Energieversorgung oder Sensorleitung notwendig, da eine für die Dialyse typische Temperaturdifferenz als Energiequelle ausgenutzt wird. Selbstverständlich können auch andere geeignete Drahtlossender und Energiequellen zum Einsatz kommen.

Die dargestellte Art der Anbringung des Sensors, des Senders und des Energiewandlers am Einwegteil hat unter anderem auch den Vorteil, dass keine Kabel an der Nadel vorhanden sind, sodass es zu keiner mechanischen Beeinträchtigung oder Gefährdung für den Zugang und zu keiner Beeinträchtigung der Bewegungsfreiheit des Patienten kommt. Die Ausführung des Sensors als Disposable bringt überdies hygienische Vorteile mit sich. Die Messung startet durch die Ausbildung der Energiequelle als thermoelektrischer Energiewandler erst, wenn das Disposable in Betrieb genommen wird. Es ist kein Einschalten notwendig und es findet kein Dauersenden des Senders statt. Eine Batterie oder ähnliches ist nicht notwendig, was sich positiv auf die Lagerungsdauer des Disposables auswirkt, die unter Umständen durch die Lebensdauer einer Batterie begrenzt sein könnte. Die Sensorik kann komplett eingegossen bzw. eingespritzt werden, sodass eine hermetische Abkapselung gegen Feuchtigkeit besteht.

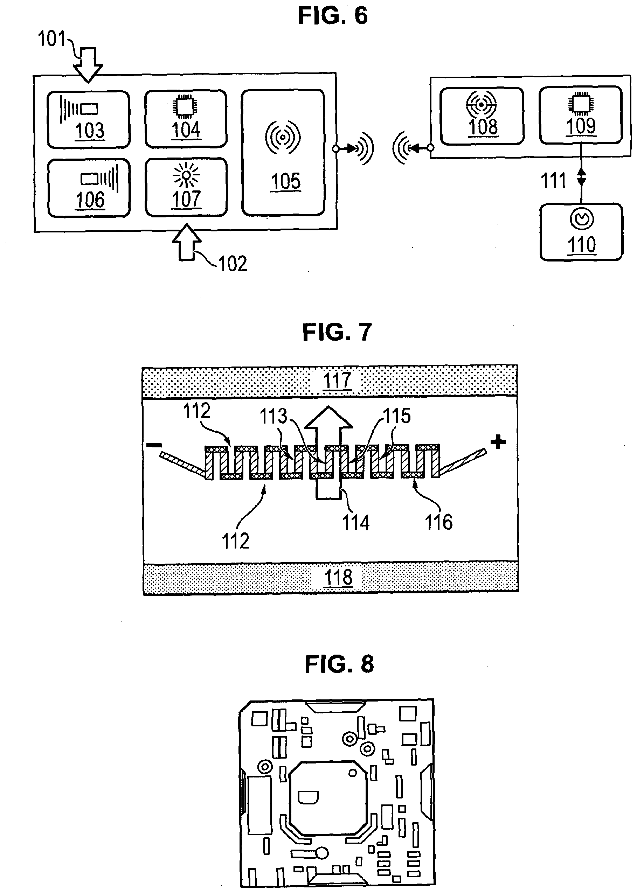

Das Funktionsprinzip der in einem Thermoenergiewandler angewandten Energiegewinnung unter Ausnutzung der für die Dialyse typischen Temperaturdifferenzen zwischen Dialysat bzw. Blut und Umgebung ist schematisch in Figur 7 gezeigt. Mit Bezugszeichen 118 ist schematisch die Lage des 37°C warmen Blutes bzw. Dialysates angedeutet, und mit Bezugszeichen 117 die beispielsweise etwa 20°C warme Umgebung. Bezugszeichen 113 zeigen p-Leiter und Bezugszeichen 115 n-Leiter. Mit Bezugszeichen 114 ist ein Wärmestrom symbolisiert. Bezugszeichen 116 zeigt metallische Brücken zwischen den p-Leitern 113 und den n-Leitern 115. Bezugszeichen 112 zeigt eine elektrische Isolierung oberhalb bzw. unterhalb der metallischen Brücken 116 und Halbleiter 113 bzw. 115.

Wenn an der Kontaktstelle zwischen n-Leiter 115 und p-Leiter 113 Wärmeenergie aufgenommen wird, so können dadurch Elektronen vom n-Leiter 115 in das energetisch höhere Leitungsband des benachbarten p-Leiters 113 gelangen. Es erfolgt ein Stromfluss also vom p-dotierten zum n-dotierten Halbleiter.

Eine Abbildung eines Thermoenergiewandler zeigt Figur 8.

In Figur 6 ist eine schematische Ablaufskizze der Messung von Daten, Energiewandlung und Übermittlung von Daten zum Einsatz in einem erfindungsgemäßen Disposable gezeigt:
Die linke Darstellung zeigt ein Sensor-Funkmodul, das disposableseitig angeordnet ist. Es umfasst eine Sensorik 106 zur Erfassung von Messdaten 102, einen Prozessor 104 zur Messdatenverarbeitung, einen Energiewandler 103 zur Gewinnung elektrischer Energie aus beispielsweise thermischer Energie 101, eine Elektronik 106 zum Energiemanagement und einen HF-Transceiver 105 als Sender.

Die rechte Seite zeigt ein maschinenseitig angeordnetes System-Funkmodul. Es umfasst einen HF-Transceiver 108 als Empfänger für die Funksignale des Senders 105 und einen Prozessor 109 zur Datenverarbeitung. Bezugszeichen 110 zeigt einen Aktor des Gerätes, der in Abhängigkeit des empfangenen Messsignals und der ausgewerteten Daten von der Gerätesteuerung angesteuert werden kann. Dabei handelt es sich beispielsweise um eine Pumpe, Heizung oder ähnliches. Übertragung und Umwandlung des Messsignals in ein Steuersignal ist mit Bezugszeichen 111 symbolisiert.

### Beispiel 2:

### Messen der Größen Druck und Temperatur am Dialysator

Die für die Behandlung wichtigen Messgrößen des Dialysatoreingangsdrucks und des Dialysatorausgangsdrucks sollen disposableseitig gemessen werden können. Das Disposable umfasst eine Blutleitung mit einer arteriellen und einer venösen Leitung, einen Dialysator und eine Dialysatleitung.

Dazu sind sowohl blutseitig als auch dialysatseitig direkt an der Eintritts- bzw. Austrittsstelle am Dialysator des Disposable Drucksensoren angeordnet. Als Drucksensoren werden kapazitive Drucksensoren SCB10H der Fa. VTI verwendet, die einen sehr niedrigem Energieverbrauch haben. Selbstverständlich können auch andere geeignete Sensoren zum Einsatz kommen.

Der Transmembrandruck kann so direkt und präzise am Dialysator bestimmt werden. Es erfolgt keine Beeinflussung der Messgrößen durch die Schlauchleitung (Druckabfälle am Schlauch), es erfolgt eine direkte Überwachung der dialysatortypischen Druckabfälle, u. a. Längsdruckabfall, über die Fasern, und es ist eine schnelle und frühe Clotting-Erkennung möglich.

Ferner soll die Temperatur des Dialysats am Dialysator gemessen werden. Als Temperatursensor wird am Dialysator disposableseitig das Mantelwiderstandsthermometer PT1000 angeordnet. Selbstverständlich können auch andere geeignete Sensoren zum Einsatz kommen. So kann der Wärmeeintag ins Blut direkt am Dialysator bestimmt werden und es erfolgt beispielsweise kein Temperaturverlust am Schlauch. Dies kann zu einer Verbesserungen im Temperaturmanagement am Dialysator genützt werden. Die gesamte Energiebilanz kann direkt am Dialysator aufgenommen werden, sodass der thermische Wirkungsgrad des Wärmetauschers Dialysator ohne systematische Messfehler bestimmt werden kann.

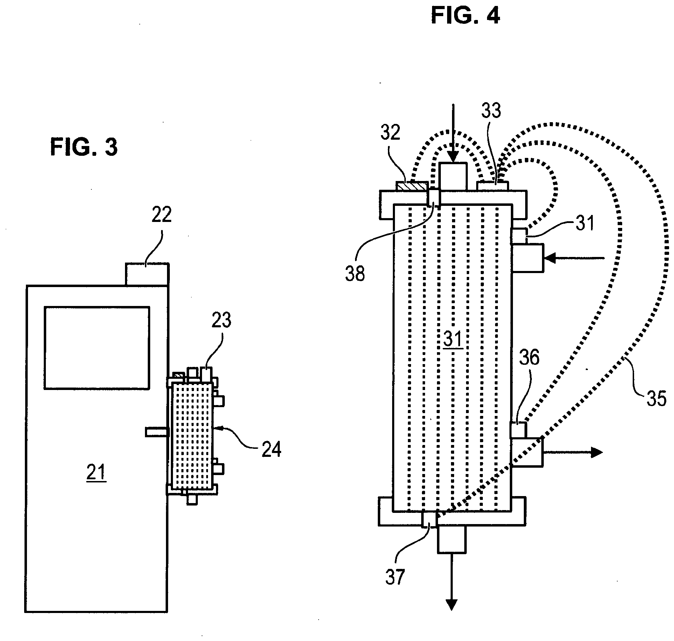

Ein derartiges System zur disposableseitigen Messdatenerfassung und drahtlosen Messdatenübermittlung ist in Figuren 3 und 4 gezeigt.

In der Darstellung gemäß Figur 3 zeigt Bezugszeichen 21 eine Dialysemaschine, an der intern oder extern die Empfangseinheit 22 angeordnet ist. Mit dem Bezugszeichen 24 wird der Dialysator symbolisiert, der mehrere Messzellen, einen Energiewandler und einen Sender 23 zur drahtlosen Übermittlung von Daten an die Empfangseinheit 22 aufweist.

In Figur 4 ist der Dialysator 24 bzw. 31 des Disposables in einer derartigen Anordnung in größerem Detail gezeigt. Wie daraus ersichtlich ist, handelt es sich dabei um einen gewöhnlichen Hohlfaserdialysator, wobei Blut in der Figur von oben nach unten durch das Innere der Hohlfasern fließt und wobei die Hohlfasern in der Dialysatorkammer von seitlich in die Dialysatorkammern zufließendem und seitlich aus der Dialysatorkammer abfließendem Dialysat umspült werden. Selbstverständlich kann der Dialysator auch im Gegenstromprinzip betrieben werden, was sogar bevorzugt ist.

Am Dialysator befinden sich mehrere Sensoren:
- Ein Drucksensor und ein Temperatursensor (zusammengefasst als Sensor 31 dargestellt) auf der Dialysatseite am Eingang des Dialysators.
- Ein Drucksensor und ein Temperatursensor (zusammengefasst als Sensor 36 dargestellt) auf der Dialysatseite am Ausgang des Dialysators.
- Ein Drucksensor und ein Temperatursensor (zusammengefasst als Sensor 37 dargestellt) auf der Blutseite am Ausgang des Dialysators.
- Ein Drucksensor und ein Temperatursensor (zusammengefasst als Sensor 38 dargestellt) auf der Dialysatseite am Eingang des Dialysators.

Mit Bezugszeichen 32 ist ein thermoelektrisches Peltier-Element bezeichnet, das den Temperaturunterschied zwischen Umgebungstemperatur, beispielsweise 20°C, und der Bluttemperatur bzw. Dialysattemperatur am Dialysator, in der Regel 37°C, zur Erzeugung von elektrischer Energie nützt.

Mit Bezugszeichen 33 ist eine Sendeeinheit bezeichnet, die die Messdaten der Sensoren 34, 36, 37 und 38 an einen maschinenseitig angeordneten Empfänger 22 übermittelt. Der Sender 33, die Sensoren 34, 36, 37 und 38 und der thermoelektrische Energiewandler 32 stehen über elektrische Verbindungen 35 in Verbindung. Anhand dieser elektrischen Verbindungen 35 werden der Sender 33 und die Sensoren 34, 36, 37 und 38 mit elektrischer Energie vom Energiewandler 32 versorgt. Ferner gelangen die Messdaten der Sensoren 34, 36, 37 und 38 anhand dieser elektrischen Verbindungen 35 zum Sender 33. Sowohl die Sensoren 34, 36, 37 und 38 als auch der Sender 33 und der Energiewandler 32 sind Teil des Disposables.

Als Sender für die Daten der Sensoren wird der ebenfalls am Disposable angeordnete Drahtlossender verwendet, der bereits im Zusammenhang mit Ausführungsbeispiel 1 beschrieben wurde. Als Energiequelle für Sensoren und Sender dient ein Standard-Peltierelement mit DC/DC Konverter, wie es bereits im Zusammenhang mit Ausführungsbeispiel 1 beschrieben wurde. Genützt wird die Temperaturdifferenz zwischen dem Dialysat (in der Regel > 35°C) und der Umgebung (in der Regel < 25°C). Selbstverständlich können auch andere geeignete Drahtlossender und Energiequellen zum Einsatz kommen.

In diesem Beispiel befinden sich keine Kabel am Dialysator, sodass es zu keiner Beeinträchtigung der Bewegungsfreiheit des Patienten kommt. Durch die Ausführung der Sensoren als Disposable bestehen hygienische Vorteile. Es sind keine externe Energieversorgung oder Sensorleitungen notwendig, da eine für die Dialyse typische Temperaturdifferenz als Energiequelle ausgenutzt wird. Die Messung startet erst, wenn das Disposable in Betrieb genommen wird. Es ist kein Einschalten notwendig und es erfolgt kein Dauersenden der Sendeeinheit. Eine Batterie oder ähnliches ist nicht notwendig, was sich positiv auf die Lagerungsdauer des Disposable auswirkt, die unter Umständen durch die Lebensdauer einer Batterie begrenzt sein könnte. Die Sensorik kann komplett eingegossen bzw. eingespritzt werden, sodass eine hermetische Abkapselung gegen Feuchtigkeit besteht.

Zusammenfassend werden also durch die disposableseitige Anordnung eines Thermogenerators am Dialysator die Druckmessung im Dialysat und/oder Blut durch disposableseitig angeordnete Drucksensoren und eine kabellose Datenübertragung in vorteilhafter Weise modifiziert. Darunter fallen unter anderem das Fehlen eines störenden Disposable zwischen Sensor und Messmedium. Die Erfindung stellt im Rahmen dieses Beispiels also ein intelligentes, batterieloses Disposable zur Verfügung.

### Beispiel 3:

### Messung von Füllstand und Temperatur direkt an einem Einwegbehälter für Dialysat

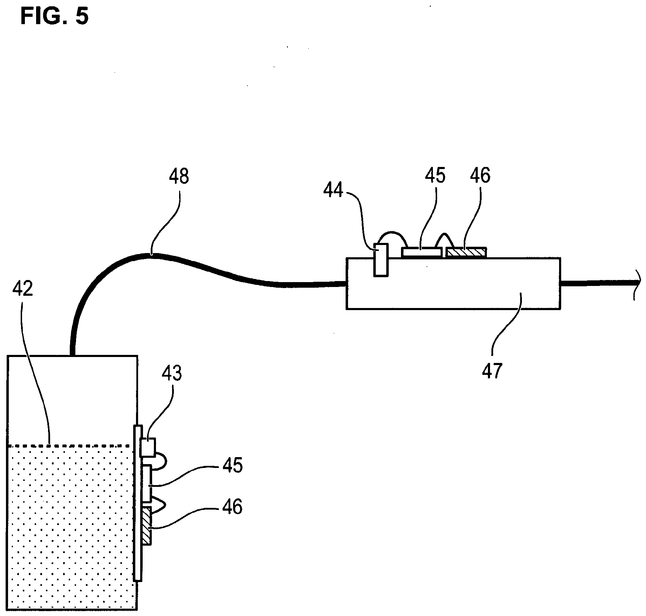

Figur 5 zeigt eine schematische Darstellung der Messung von Füllstand und Temperatur an einem Einwegbehälter anhand disposableseitig angeordneter Sensoren, Energiewandler und Drahtlossender.

In dieser Figur 5 ist der Einwegbehälter links unten gezeigt und der ebenfalls zum Disposable gehörige Schlauch 48 rechts oben.

Mit dem Bezugszeichen 43 ist ein disposableseitig angeordneter Levelsensor bezeichnet, der den Füllstand 42 des Behälters erfasst. Mit dem Bezugszeichen 44 ist ein disposableseitig angeordneter Temperatursensor 44 (PT1000) gezeigt, der die Temperatur des Dialysats mit zwei Tüllen in der kleinen Kammer 47 erfasst, die an der Schlauchleitung 48 angeordnet ist. Selbstverständlich kann das Disposable auch lediglich den Füllstandssensor 43 oder den Temperatursensor 44 umfassen.

Mit Bezugszeichen 46 sind thermoelektrische Peltier-Elemente bezeichnet, die den Temperaturunterschied zwischen Umgebungstemperatur (beispielsweise 20°C) und der Temperatur des Dialysats (typischerweise 37°C) zur Erzeugung von elektrischer Energie nützt.

Mit Bezugszeichen 45 sind Sendeeinheiten bezeichnet, die die Messdaten der Sensoren 43 und/oder 44 an einen maschinenseitig angeordneten Empfänger übermitteln.

Die Sender 45, die Sensoren 43 und/oder 44 und die thermoelektrischen Energiewandler 46 stehen so in Verbindung, dass ein Sender 45 und ein Sensor 43 bzw. 44 mit elektrischer Energie von einem dazugehörigen Energiewandler 46 versorgt werden. Ferner gelangen die Messdaten eines jeweiligen Sensors 43 bzw. 44 zum jeweiligen Sender 45. Sowohl die Sensoren 43 und/oder 44 als auch die Sender 45 und die Energiewandler 46 sind Teil des Disposables.

Als Sender und Energiewandler werden die im Zusammenhang mit Ausführungsbeispiel 1 erläuterten Bauteile verwendet.

### Beispiel 4:

### Niveau- und/oder Druck- und/oder Leitfähigkeits- und/oder Temperaturmessung in der Hydraulik

In diesem Beispiel wird ein Thermogenerator maschinenseitig an der Hydraulik angeordnet. Es erfolgt eine Messwerterfassung an der Hydraulik durch einen aufgrund des Thermogenerators energieautarken Sensor und eine kabellose Datenübertragung durch einen aufgrund des Thermogenerators energieautarken und mit dem Sensor verbundenen Senders. Dieses Konzept der Messdatenerfassung lässt sich im Wesentlichen auf jede Messstelle und auf jeden gewünschten Messwert in der Hydraulik anwenden.

Die Vorteile umfassen das Entfallen des Bedarfs für Kabel oder Batterie. Die autarke Messeinheit ist platzsparend, einfach zu montieren und kann einfach gewartet werden.

Als Sender und Energiewandler werden im Beispiel die im Zusammenhang mit Ausführungsbeispiel 1 erläuterten Bauteile verwendet.

### Beispiel 5:

### Niveau- und/oder Druck- und/oder Leitfähigkeits- und/oder Temperaturmessung in einer Wasseraufbereitungsanlage für Permeat

In diesem Beispiel wird ein Thermogenerator maschinenseitig an der Wasseraufbereitungsanlage für Permeat, beispielsweise an einer Rohrleitung der Wasseraufbereitungsanlage angeordnet. Es erfolgt eine Messwerterfassung durch einen aufgrund des Thermogenerators energieautarken Sensor und eine kabellose Datenübertragung durch einen aufgrund des Thermogenerators energieautarken und mit dem Sensor verbundenen Senders.

In der Dialysetechnik werden Wasseraufbereitungsanlagen für die Permeatherstellung verwendet. Diese Systeme werden immer häufiger einer regelmäßigen Desinfektion/Reinigung mit Heißwasser unterzogen. Dabei muss der Nachweis erbracht werden, dass in allen Bereichen des Rohrleitungssystems die für eine Reinigung benötigte Temperatur erreicht wird. Hierbei sind im besonderen Maße die Stellen in außen liegenden Bereichen des Systems relevant. Somit ist häufig die örtliche Distanz zwischen der Messstelle und der Auswerteeinheit (Dialysegerät/RO-Anlage) groß. Die autarke Messeinheit ist daher besonders vorteilhaft.

Während der thermischen Desinfektion ist der Temperaturunterschied zwischen Rohrleitung/Maschinenteil und der Umgebung groß. Die Energiegewinnung mit Hilfe eines Thermogenerators ist daher besonders effizient.

Als Sender und Energiewandler werden im Beispiel die im Zusammenhang mit Ausführungsbeispiel 1 erläuterten Bauteile verwendet.

## Patentansprüche

1. Einwegartikel zur Verwendung in der Dialysebehandlung, umfassend:
einen Sensor (13) zur Ermittlung eines die Dialyse betreffenden Messwertes, und
einen Sender (14), der dazu ausgebildet ist, eine drahtlose Übertragung des Messwertes an einen außerhalb des Einwegartikels befindlichen Empfänger (4) zu ermöglichen, wobei
der Einwegartikel ein extrakorporaler Blutkreislauf für die Hämodialyse ist, der eine Blutleitung mit einer arteriellen Leitung und einer venösen Leitung (16) umfasst, und
der Sensor ein Drucksensor (13) ist, der in der venösen Leitung (16) des extrakorporalen Blutkreislaufs angeordnet ist,
**gekennzeichnet durch**
einen thermoelektrischen Energiewandler (15) zur Bereitstellung elektrischer Energie, wobei
der Drucksensor (13) direkt an einem Katheter (12) zum Anschließen der venösen Leitung (16) an einen Patienten angeordnet ist,
der Sender (14), der Drucksensor (13) und der thermoelektrische Energiewandler (15) über elektrische Verbindungen (17) in Verbindung stehen,
anhand dieser elektrischen Verbindungen (17) der Sender (14) und der Drucksensor (13) mit elektrischer Energie vom Energiewandler (15) versorgt werden, und
der vom Drucksensor (14) ermittelte Messwert anhand dieser elektrischen Verbindungen (17) zum Sender (14) gelangt.

2. Einwegartikel nach Anspruch 1, wobei der thermoelektrische Energiewandler (15) ein Peltier-Element ist.

3. Einwegartikel nach einem der vorhergehenden Ansprüche, wobei der extrakorporale Blutkreislauf für die Hämodialyse ferner einen Dialysator und eine Dialysatleitung umfasst.

4. Einwegartikel nach einem der vorhergehenden Ansprüche, wobei der thermoelektrische Energiewandler (15) in der venösen Leitung (16) des extrakorporalen Blutkreislaufs und vorzugsweise nahe des Katheters (12) angeordnet ist.

5. Einwegartikel nach einem der Ansprüche 3 oder 4, wobei im extrakorporalen Blutkreislauf mindestens zwei Drucksensoren so angeordnet sind, dass der Transmembrandruck am Dialysator gemessen werden kann.

6. Dialysegerät mit einem Einwegartikel nach einem der vorhergehenden Ansprüche.

7. Dialysegerät nach Anspruch 6 ferner mit einem Empfänger für Signale von im Einwegartikel angeordneten Sendern.

## Claims

1. Disposable article for use in dialysis treatment, comprising:
a sensor (13) for determining at least one measured value relating to the dialysis,
and
a transmitter (14), which is arranged so that it enables a wireless transmission of the measured value to a receiver (4) arranged outside the disposable article, wherein
the disposable article is an extracorporeal blood circuit for hemodialysis which comprises a blood line having an arterial line and a venous line (16), and
the sensor is a pressure sensor (13), which is arranged in the venous line (16) of the extracorporeal blood circuit,
**characterized by**
a thermoelectric energy converter (15) for providing electric energy, wherein
the pressure sensor (13) is arranged directly on a catheter (12) for connecting the venous line (16) to a patient,
the transmitter (14), the pressure sensor (13) and the thermoelectric energy converter (15) communicate via electric connections (17),
the transmitter (14) and the pressure sensor (13) are supplied with electric energy from the energy converter (15) using these electric connections (17), and
the measured value determined by the pressure sensor (14) reaches the transmitter (14) using these electric connections.

2. Disposable article in accordance with claim 1, wherein the thermoelectric energy converter (15) is a Peltier element.

3. Disposable article in accordance with one of the preceding claims, wherein the extracorporeal blood circuit for hemodialysis further comprises a a dialyzer and/or a dialysate line.

4. Disposable article in accordance with one of the preceding claims, wherein the thermoelectric energy converter (15) is arranged in the venous line (16) of the extracorporeal blood circuit and preferably close to the catheter (12).

5. Disposable article in accordance with one of claims 3 or 4, wherein at least two pressure sensors are arranged in the extracorporeal blood circuit such that the transmembrane pressure can be measured at the dialyzer.

6. Dialyzer having at least one disposable article in accordance with one of the preceding claims.

7. Dialyzer in accordance with claim 6, further having least one receiver for signals from transmitters arranged in the disposable article.

## Revendications

1. Article à usage unique destiné à être utilisé dans le traitement par dialyse, comprenant :
un capteur (13) servant à déterminer une valeur de mesure concernant la dialyse, et
un émetteur (14), qui est configuré pour permettre une transmission sans fil de la valeur de mesure à un récepteur (4) se trouvant à l'extérieur de l'article à usage unique, dans lequel
l'article à usage unique est un système de circulation sanguine extracorporelle pour l'hémodialyse, qui comprend un conduit sanguin avec un conduit artériel et un conduit veineux (16), et
le capteur est un capteur de pression (13), qui est disposé dans le conduit veineux (16) du système de circulation sanguine extracorporelle,
**caractérisé par**
un convertisseur d'énergie (15) thermoélectrique servant à fournir une énergie électrique, dans lequel
le capteur de pression (13) est disposé directement au niveau d'un cathéter (12) servant à raccorder le conduit veineux (16) à un patient,
l'émetteur (14), le capteur de pression (13) et le convertisseur d'énergie (15) thermoélectrique communiquent par l'intermédiaire de liaisons électriques (17),
à l'aide desquelles liaisons électriques (17) l'émetteur (14) et le capteur de pression (13) sont alimentés en énergie électrique du convertisseur d'énergie (15), et
la valeur de mesure déterminée par le capteur de pression (13) parvient à l'aide desdites liaisons électriques (17) à l'émetteur (14).

2. Article à usage unique selon la revendication 1, dans lequel le convertisseur d'énergie (15) thermoélectrique est un élément Peltier.

3. Article à usage unique selon l'une quelconque des revendications précédentes, dans lequel le système de circulation sanguine extracorporelle pour l'hémodialyse comprend en outre un dialyseur et un conduit de dialysat.

4. Article à usage unique selon l'une quelconque des revendications précédentes, dans lequel le convertisseur d'énergie (15) thermoélectrique est disposé dans le conduit veineux (16) du système de circulation sanguine extracorporelle et de préférence à proximité du cathéter (12).

5. Article à usage unique selon l'une quelconque des revendications 3 ou 4, dans lequel au moins deux capteurs de pression sont disposés dans le système de circulation sanguine extracorporelle de telle manière que la pression transmembranaire peut être mesurée au niveau du dialyseur.

6. Appareil de dialyse avec un article à usage unique selon l'une quelconque des revendications précédentes.

7. Appareil de dialyse selon la revendication 6 en outre avec un récepteur pour des signaux d'émetteurs disposés dans l'article à usage unique.
